(19) Europäisches Patentamt / European Patent Office / Office européen des brevets

(11) **EP 3 907 739 A1**

(12) **EUROPEAN PATENT APPLICATION**
published in accordance with Art. 153(4) EPC

(43) Date of publication:
**10.11.2021 Bulletin 2021/45**

(51) Int Cl.:
*G16B 20/40* (2019.01)   *G16B 30/00* (2019.01)
*G16B 50/00* (2019.01)

(21) Application number: **19907370.1**

(22) Date of filing: **20.11.2019**

(86) International application number:
**PCT/KR2019/015927**

(87) International publication number:
**WO 2020/141722 (09.07.2020 Gazette 2020/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.01.2019 KR 20190001306**

(71) Applicant: **Theragen Genomecare Co., Ltd.
Gyeonggi-do 16229 (KR)**

(72) Inventor: **KIM, Sun Shin
Yongin-si, Gyeonggi-do 17003 (KR)**

(74) Representative: **HGF
1 City Walk
Leeds LS11 9DX (GB)**

(54) **METHOD FOR DETERMINING FETAL FRACTION IN MATERNAL SAMPLE**

(57)    Provided are a method of determining a fetal fraction and a computer readable medium, in which a program to be applied for performing the method is recorded. According to the method, when the fetal fraction is estimated in an optimal bin different from the predetermined chromosome bin (50 kb), the fetal fraction may be more accurately determined. Therefore, the fetal fraction may be measured with higher accuracy using the same training sample size.

FIG. 8C

EP 3 907 739 A1

## Description

## Technical Field

The present invention relates to a method of determining a fetal fraction in a biological sample derived from a pregnant woman and software related to the method.

## Background Art

Prenatal diagnosis refers to diagnosis of the presence or absence of diseases in a fetus before the fetus is born. Prenatal diagnosis is largely classified into an invasive diagnostic test and a non-invasive prenatal diagnostic test (NIPT). Invasive diagnostic tests include, for example, chorionic villus sampling, amniocentesis, and cordocentesis. Invasive diagnostic tests may cause abortion or diseases by impacting a fetus during the examination process, and therefore, non-invasive diagnostic tests have been developed.

Recently, it has been demonstrated that non-invasive diagnosis of fetal chromosomal aneuploidy is feasible by massively parallel sequencing of DNA molecules in the plasma of pregnant women. Fetal DNA is detectable in maternal plasma and serum from the seventh week of gestation, and the amount of fetal DNA in maternal blood increases as pregnancy progresses. The ratio of fetal DNA among cell-free DNA (cfDNA) isolated from maternal blood is referred to as a fraction of fetal nucleic acids or a fetal fraction. The smaller the fetal fraction, the higher the diagnostic error of chromosomal aneuploidy, and thus the fetal fraction may affect the accuracy of the non-invasive prenatal diagnostic test.

Regarding a method of measuring the fetal fraction, a SNP-based method has high accuracy, but there is a problem in that a cost of the genotyping is high (Jiang P. et al., Bioinformatics, 2012, vol.28, pp.2883-2890), and a Y chromosome-based method is simple but has a problem in that it only applies to male fetuses (Hudecova I. et al., PloS one, 2014, vol.9, p.e88484). In addition, machine learning had an advantage of no additional costs, but there are problems in that it requires a large amount of training samples corresponding to about 25,000 training samples and high computing power, and it can produce different results depending on several factors such as the number of sequence information (reads) of nucleic acid fragments (Kim et al., Prenatal diagnosis, 2015, vol.35, pp.810-815; Johansen P. et al., Prenatal diagnosis, 2016, vol.36, pp.530-536).

Therefore, there is a need to develop a method for estimating the fetal fraction with excellent accuracy with no additional cost, as well as a small amount of training samples and low computing power.

## Detailed Description of Invention

### Technical Problem

Provided is a method of determining a fetal fraction.

Provided is a computer readable medium, in which a program to be applied for performing the method of determining a fetal fraction is recorded.

### Solution to Problem

According to an aspect of the present disclosure, provided is a method of determining a fetal fraction in a biological sample of a pregnant woman, wherein the method comprises

generating test data by obtaining sequence information (reads) of a plurality of nucleic acid fragments from a biological sample of a pregnant woman;
setting a chromosome bin divided by constant bin based on a reference chromosome;
generating a parameter from the training data;
measuring a fetal fraction from the test data using the generated parameter;
selecting a bin having a high correlation between the measured fetal fraction and the standard fetal fraction by repeating the steps of generating the parameter and measuring the fetal fraction while increasing the bin size based on the reference chromosome;
generating a second parameter having an increased correlation between the measured fetal fraction and the standard fetal fraction while increasing the size of the training data in the selected bin; and
measuring a fetal fraction from the test data using the generated second parameter.

As used herein, the term "fetal fraction" or "fraction of fetal nucleic acids" refers to an amount of fetal nucleic acids among nucleic acids isolated from a biological sample of a pregnant woman. The fetal fraction may be a concentration, a relative ratio, or an absolute amount of the fetal nucleic acids. The fetal nucleic acids may be nucleic acids derived from a fetal placenta trophoblast.

The method includes generating test data by obtaining sequence information (reads) of a plurality of nucleic acid fragments from a biological sample of a pregnant woman.

The biological sample may be blood, plasma, serum, urine, saliva, mucus, sputum, feces, tears, or a combination thereof. The biological sample is, for example, plasma of the peripheral blood. The biological sample may contain nucleic acids derived from a mother of a fetus and nucleic acids derived from a fetus. The nucleic acids may be cell-free DNA (cfDNA).

The generation of test data by obtaining sequence information of a plurality of nucleic acid fragments may include isolating cell-free DNAs (cfDNAs) from the biological sample. The isolating of nucleic acids or cell-free DNAs from the biological sample may be performed by a method known to those skilled in the art. The isolated nucleic acid fragments may be about 10 bp (base pairs) to about 2000 bp, about 15 bp to about 1500 bp, about 20 bp to about 1000 bp, about 20 bp to about 500 bp, about 20 bp to about 200 bp, or about 20 bp to about 100 bp in length.

The generation of test data by obtaining sequence information of a plurality of nucleic acid fragments from a biological sample obtained from a pregnant woman may include performing massively parallel sequencing of the isolated nucleic acids. The term "massively parallel sequencing" may be used interchangeably with next-generation sequencing (NGS) or second-generation sequencing. The massively parallel sequencing refers to a technique in which millions of nucleic acid fragments are sequenced simultaneously. The massively parallel sequencing may be performed in parallel by, for example, 454 platform (Roche), GS FLX titanium, Illumina MiSeq, Illumina HiSeq, Illumina Genome Analyzer, Solexa platform, SOLiD System (Applied Biosystems), Ion Proton (Life Technologies), Complete Genomics, Helicos Biosciences Heliscope, single molecule real-time sequencing technology (SMRT™) of Pacific Biosciences, or a combination thereof.

The obtained sequence information of the nucleic acid fragments may be also referred to as reads.

The term "sequencing coverage" is also referred to as a sequencing depth, which denotes a number of reads that include a given nucleotide in the sequence reconstructed by massively parallel sequencing. In order to distinguish between sequencing errors and actual nucleotide polymorphism, an accuracy of sequencing needs to be increased by sequencing individual genomes several times. The sequencing coverage may be an average number of sequence information that is subjected to be sequenced. The sequencing coverage may be computed from Equation of (length of reads x number of reads)/haploid genome length. The sequencing coverage of the obtained sequence information may be about 0.001 to about 3.5, about 0.003 to about 3.5, about 0.005 to about 3.5, about 0.007 to about 2.5, about 0.01 to about 2.5, about 0.015 to about 1.5, about 0.02 to about 1.5, about 0.025 to about 1.5, about 0.01 to about 1.2, about 0.01 to about 1.2, or about 0.01 to about 1.0.

The method comprises setting a chromosome bin divided by constant bin based on a reference chromosome.

The reference chromosome may be a human reference chromosome. The human reference genome may be hg18 or hg19. The sequence information mapped to only one genome location in the human reference genome may be assigned as a unique sequence information. Based on the assigned unique sequence number, the sequence information of the nucleic acid fragments may be assigned to a chromosomal location. The chromosomal location may be within a consecutive region of a chromosome which is about 5 kb or more, about 10 kb or more, about 20 kb or more, about 50 kb or more, about 100 kb or more, about 1000 kb or more, or 2000 kb or more in length. The chromosomal location may be on a single chromosome.

The method may further include excluding bins with a low confidence level for sequence information from subjects of analysis by examining depth distribution of the sequence information of the nucleic acid fragments assigned to the chromosome at each bin, after assigning the sequence information of the nucleic acid fragments to the chromosome.

The method may further include excluding bins with a low confidence level for sequence information from subjects of analysis by examining depth distribution of the sequence information of the nucleic acid fragments assigned to the chromosome at each bin, after assigning the sequence information of the nucleic acid fragments to the chromosome. The bin may be a genomic region which is set in units of about 5 kb to about 260000 kb. For example, the bin may be a region which is set in units of about 100 kb to about 200000 kb, about 1000 kb to about 150000 kb, or about 10000 kb to about 100000 kb. By setting the bin, grouping of the sequence information depth and GC content of the nucleic acid fragments assigned to the chromosome may be performed and statistical analysis may be possible.

The excluding of bins with a low confidence level for sequence information from subjects of analysis may include removing mismatches, removing the sequence information assigned to a plurality of sites, removing duplicated sequence information, or a combination thereof. In order to exclude bins with a low confidence level for sequence information from subjects of analysis, quality filtering, trimming, perfect match, removal of multi-assigned sequences, removal of PCR duplicated reads, or a combination thereof may be performed. The perfect match is a process of selecting only nucleotide sequences that perfectly match when mapped to a human reference genome. Since multi-assigned sequences are likely to be a repeated sequence region, the multi-assigned sequences may be removed from the obtained sequence information. The removal of PCR duplicated reads is for removing more amplified regions due to errors during sequencing.

In addition, for statistical analysis, statistically significant results may be obtained by choosing groups with small deviation. Further, no depth region is usually the N-region of the chromosome, which may be removed from the subjects of analysis.

The method may further include performing locally weighted scatterplot smoothing (LOWESS or LOESS) regression analysis of the sequence information of the nucleic acid fragments according to the following Equation 1 to reduce GC content bias, after assigning the sequence information of the nucleic acid fragments to the chromosome:

$$Rf_{ij'} = RC_{ij} / \sum_{j=1}^{j=K} RC_{ij}$$ (Equation 1).

The GC content bias is also referred to as GC bias. The GC content bias refers to a difference between actual GC content of the sequenced sequence information and predicted GC content based on the reference sequence. The number of reads in each bin may become unequal due to the GC content bias.

In the above Equation 1, $Rf_{ij'}$ represents a fraction of reads on chromosome bin j in adjusted sample i, $RC_{ij}$ represents a adjusted number of unique reads on chromosome bin j in sample i, and K is the maximum value of the bin divided in the autosomal chromosome.

The method may include calculating the GC content and fraction of the sequence information (fraction of reads: Rf) of the nucleic acid fragments on the chromosome bin to the number of the nucleic acid fragments, based on the sequence information of the nucleic acid fragments assigned to the chromosome.

The fraction of the sequence information (fraction of reads: Rf) of the nucleic acid fragments is also referred to as a read ratio. The Rf refers to a ratio of the number of the nucleic acid fragments for the test sample and chromosome bin to the number of the nucleic acid fragments to be analyzed.

The GC content represents a proportion (%) of guanine (G) and cytosine (C) in bases constituting DNA. The GC content may be calculated from Equation of GC content = (G+C) / (A+T+G+C).

The chromosome bin may be a bin which is set in units of about 5 kb to about 260000 kb. For example, the chromosome bin is a bin which is set in units of about 100 kb to about 200000 kb, about 1000 kb to about 150000 kb, about 10000 kb to about 100000 kb, about 100 kb to about 150000 kb, about 100 kb to about 100000 kb, about 100 kb to about 10000 kb, about 100 kb to about 1000 kb, about 100 kb to about 900 kb, about 100 kb to about 800 kb, about 200 kb to about 800 kb, about 300 kb to about 800 kb, about 300 kb to about 700 kb, about 300 kb to about 600 kb, about 300 kb to about 500 kb, about 300 kb to about 400 kb, about 400 kb to about 800 kb, about 500 kb to about 800 kb, about 600 kb to about 800 kb, or about 700 kb to about 800 kb.

The method comprises generating a parameter from the training data.

The term "training data" refers to a dataset used for machine learning or learning in machine learning models. The training data may be a fetal sample of the same sex as a test data or sequence information derived from a fetal sample of a different sex.

The parameter may be a read count, a read size (or read length), or a combination thereof. The read count may be a ratio of the read count. The read size may be a ratio of the read size. The size of the sequence information, i.e., reads, may be different between a fetus and a mother. Since it is known that the maternal read size is larger than the fetal read size, a ratio of the read size within each bin may be expressed as a characteristic of the fetal sequence information. In this regard, the criterion for distinguishing the length of the sequence information fragment of the mother and the fetus may be about 50 bp to about 300 bp, about 50 bp to about 250 bp, about 50 bp to 200 bp, about 50 bp to about 150 bp, or about 50 bp to about 100 bp. The number of the maternal reads and the fetal reads may have different distributions depending on the location of each reference chromosome. Since the fetal reads are derived from the maternal placenta, more reads may be distributed than the maternal reads in a specific region of the chromosome.

The generation of a parameter from the training data may be the training of the training data by a machine learning method. The training of the training data may be performed by a multivariate regression model, a deep learning algorithm, or a combination thereof. The method may be performed using an open source software library of the R package cv.glmnet, Tensorflow, or a combination thereof.

The generation of a parameter from the training data may comprise measuring the fetal fraction of the training data; and generating a parameter from the measured fetal fraction.

The generation of a parameter from the training data may be performed according to the following multivariate regression equation:

$$Y = \sum_{i=0}^{i=K} \beta_i X_i + e$$

In the above equation, Y is the fetal fraction measured based on the SNP in the case of a female fetus, and the fetal fraction measured based on the SNP or with the Y chromosome in the case of a male fetus. $\beta_0$ is the intercept. $\beta_{1\text{-}K}$ is the regression coefficient. K is the maximum value of the chromosome bin of the autosomal chromosome. $X_i$ is the normalized value of the read-count ratio or the read-size ratio in bin i. e is a residual standard deviation.

In the above equation, when a female fetal sample is used as training data, Y may be the fetal fraction measured based on the SNP in the case of a female fetus. when a male fetal sample is used as training data, Y may be the fetal fraction measured based on the SNP or with the Y chromosome in the case of a male fetus. In the above equation, $\beta_0$ may be the intercept, $\beta_{1\text{-}K}$ may be the regression coefficient, K may be the maximum value of the chromosome bin of the autosomal chromosome, $X_i$ may be the normalized value of the read-count ratio or the read-size ratio in bin i, and e may be a residual standard deviation. In order to estimate the intercept and the coefficient, cv.glmnet that supports cross-validation may be run in the R package.

The fetal fraction may be an average value of the fetal fraction measured using the read count and the fetal fraction measured using the read size.

According to another aspect of the present disclosure, provided is a method of determining a fetal fraction in a biological sample of a pregnant woman, wherein the method comprises

generating test data by obtaining sequence information of a plurality of nucleic acid fragments from a biological sample of a pregnant woman;
setting a chromosome bin in units of 100 kb to 900 Kb based on a reference chromosome;
generating a read count, a read size, or a combination thereof as a parameter from the training data; and
measuring a fetal fraction from the test data using the generated parameter.

When the parameter in the method is a read count, the chromosome bin may be a bin which is set in units of about 100 kb to about 800 kb, about 100 kb to about 700 kb, about 100 kb to about 600 kb, about 100 kb to about 400 kb, about 100 kb to about 300 kb, about 200 kb to about 800 kb, or about 300 kb to about 800 kb.

When the parameter in the method is a read size, the chromosome bin may be a bin which is set in units of about 200 kb to about 900 kb, about 300 kb to about 900 kb, about 400 kb to about 900 kb, about 500 kb to about 900 kb, about 600 kb to about 900 kb, about 700 kb to about 900 kb, about 700 kb to about 800 kb, or about 800 kb to about 900 kb.

According to another aspect of the present disclosure, provided is a computer readable medium in which a program to be applied for performing the method according to an aspect is recorded. The computer readable medium includes a system including a computer readable medium.

**Effects of Invention**

According to the method of determining a fetal fraction according to an aspect and a computer readable medium, in which a program to be applied for performing the method is recorded, when the fetal fraction is estimated in an optimal bin different from the predetermined chromosome bin (50 kb), the fetal fraction may be more accurately determined. Therefore, the fetal fraction may be measured with higher accuracy using the same training sample size.

**Brief Description of Drawings**

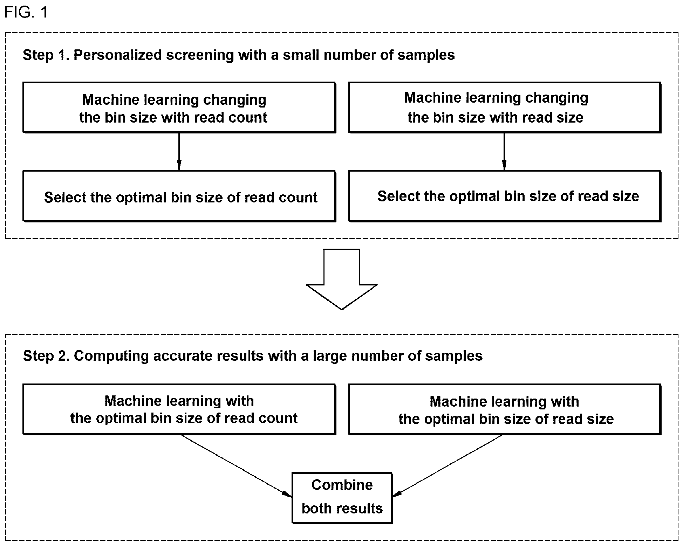

FIG. 1 is a schematic diagram of a method of measuring a fetal fraction by personalized machine learning according to an aspect.

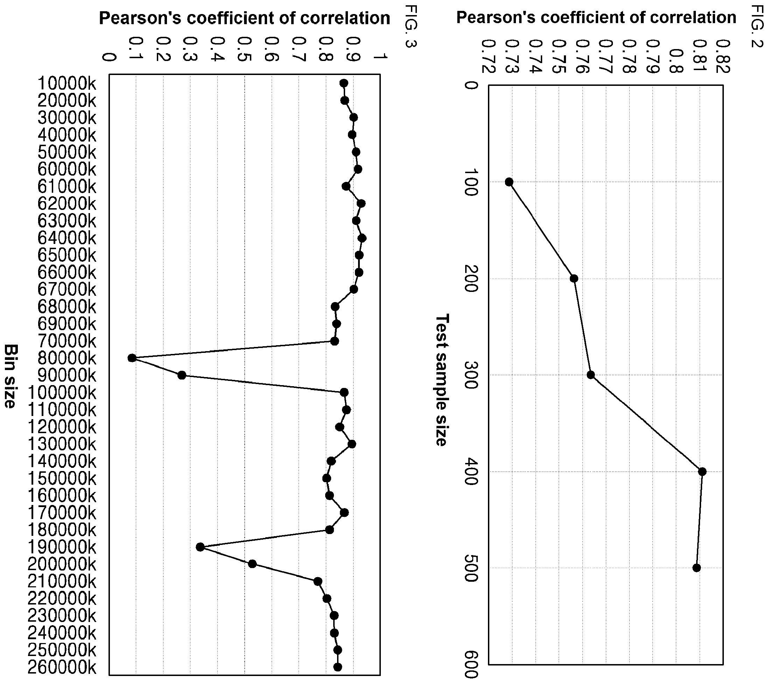

FIG. 2 is a graph showing the correlation distribution between the test sample size and the standard fetal fraction measured using SeqFF (x-axis: test sample size, y-axis: Pearson's coefficient of correlation between the test sample size and the standard fetal fraction).
FIG. 3 is a graph showing the Pearson correlation distribution in which the fetal fraction measured according to each autosomal chromosome divided bin size using 1000 male fetal training samples and 117 male fetal test samples is compared with the Y chromosome-based fetal fraction (x-axis: bin size, y-axis: Pearson's coefficient of correlation).

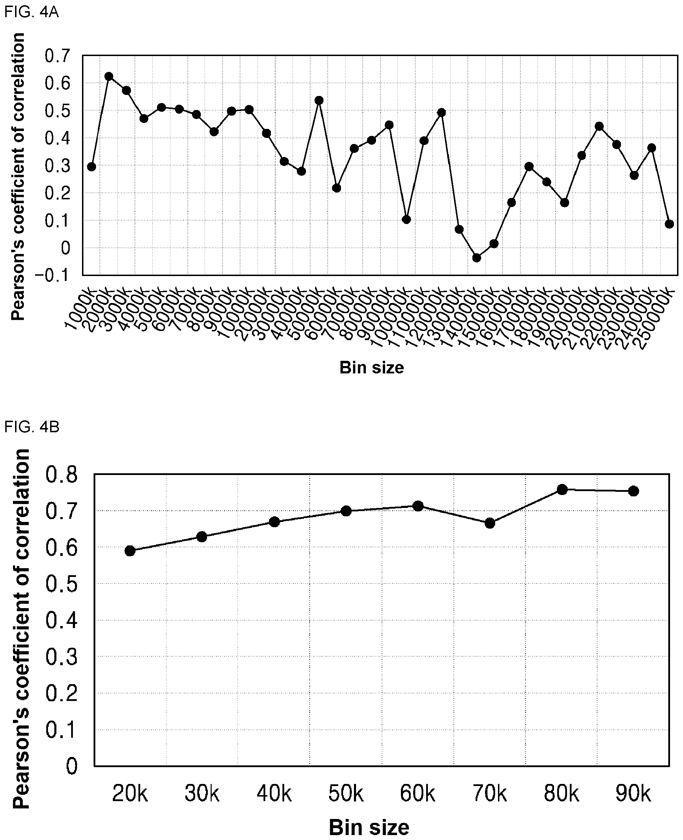

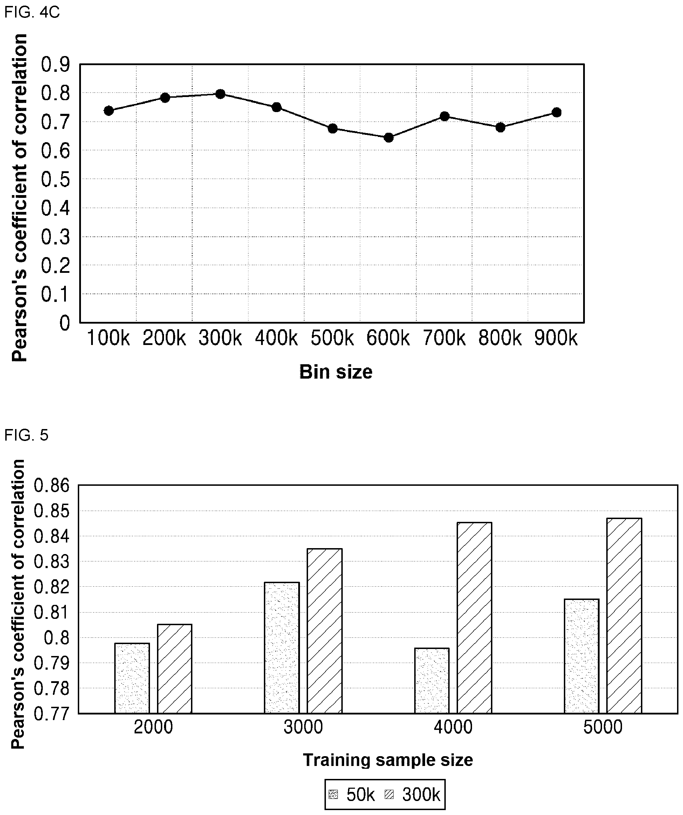

FIGs. 4A, 4B, and 4C are graphs showing the Pearson correlation distribution in which the female fetal fraction measured according to each different autosomal chromosome divided bin using 1000 male fetal training samples and 45 female fetal test samples is compared with the SNP-based standard fetal fraction (x-axis: bin size, y-axis: Pearson's coefficient of correlation).

FIG. 5 is a graph showing the Pearson correlation distribution when the training sample size is increased from 2000 to 5000 and the chromosome bin size is 50 kb or 300 kb (x-axis: training sample size, y-axis: Pearson's coefficient of correlation).

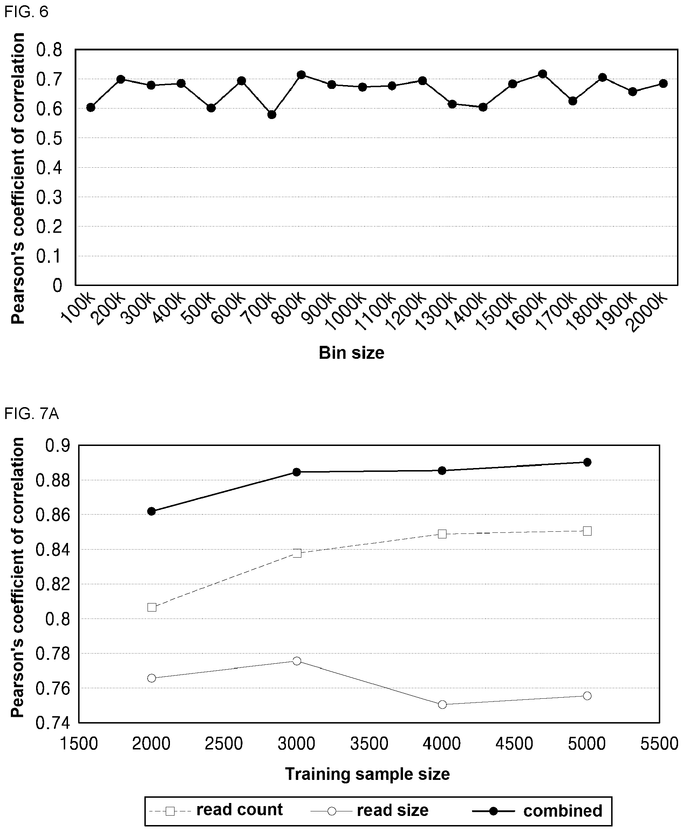

FIG. 6 is a graph showing the Pearson correlation distribution in which the fetal fraction estimated using the read-size ratio is compared with the SNP-based standard fetal fraction (x-axis: bin size, y-axis: Pearson's coefficient of correlation).

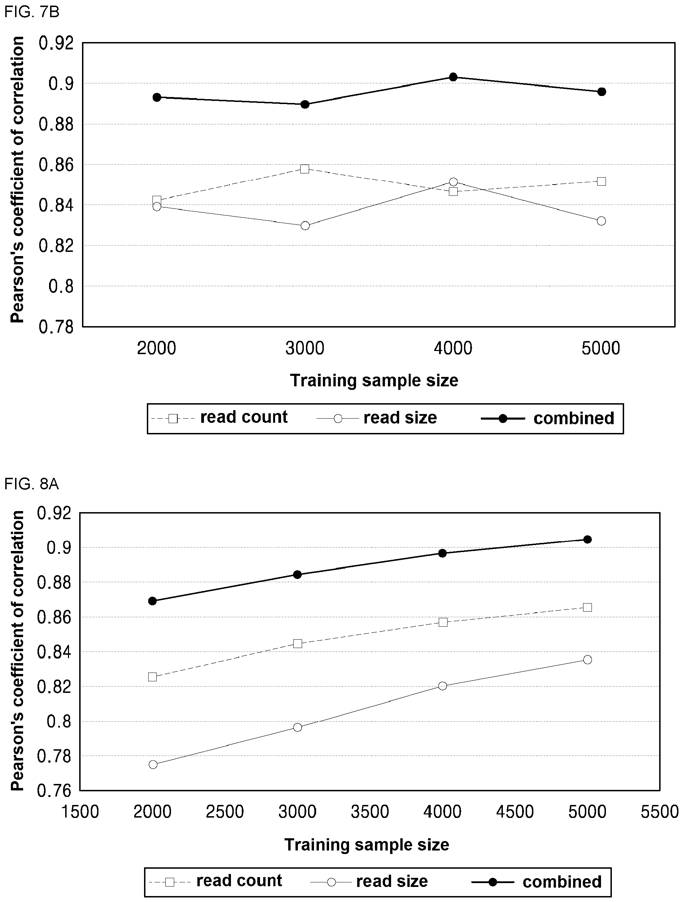

FIGs. 7A and 7B are graphs showing the Pearson correlation distribution for the fetal fraction calculated using the read count, the fetal fraction calculated using the read length, and the average value of the two fetal fractions while increasing the training sample size when the size of the bins divied in autosomal chromosome is 300 kb (FIG. 7A: 45 female fetal test samples, FIG. 7B: 45 male fetal test samples, x-axis: training sample size, y-axis: Pearson's coefficient of correlation).

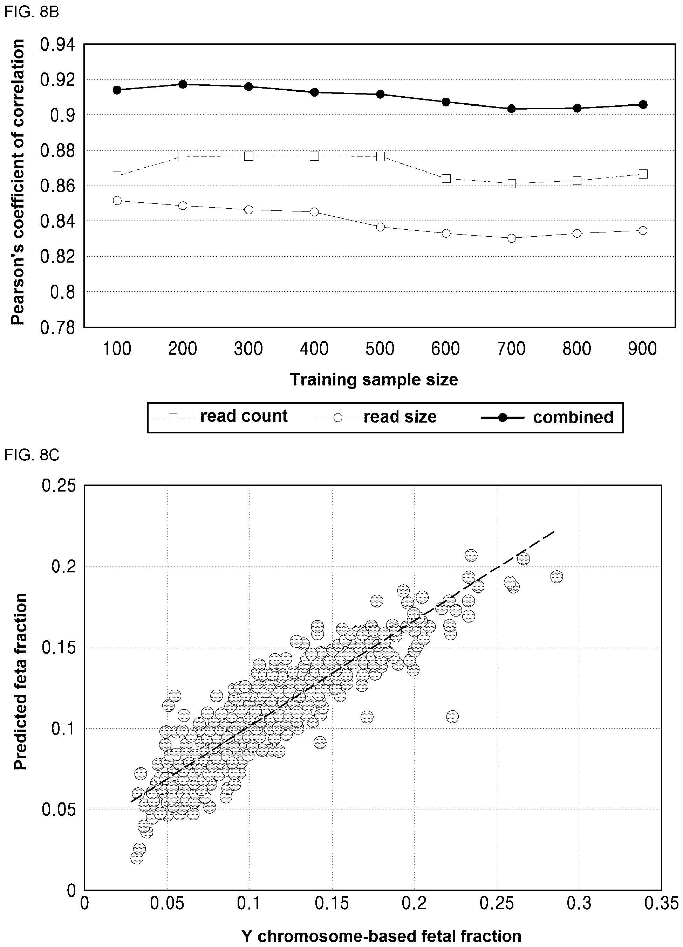

FIG. 8A is a graph showing the Pearson correlation distribution computed when using 900 male fetal test samples and increasing the training sample size, FIG. 8B is a graph showing the correlation distribution computed when using 5,000 training samples and increasing the test sample size, and FIG. 8C is a graph showing the correlation between the fetal fraction estimated using 5,000 training samples and 900 test samples and the Y chromosome-based fetal fraction.

**Mode for Carrying out the Invention**

Hereinafter, the present invention will be described in more detail with reference to Examples. However, these examples are for illustrative purposes of the present invention only, and the scope of the present invention is not limited to these examples.

**Example 1. Measurement of fetal fraction**

**1. Sample preparation and determination of baseline fetal fraction**

The serum samples were prepared from a total of 5,990 pregnant women (5,945 male fetuses and 45 female fetuses). A semiconductor sequencing was performed on the obtained serum. The fetal fraction for 5,900 male fetuses was measured by a Y chromosome-based method (Hudecova, et al., PLoS One 2014;9 (2):e88484). In addition, the fetal fraction for the serum of 90 pregnant women (45 female fetuses and 45 male fetuses) was measured using SNPs, and for this, the genotype was analyzed using the Affymetrix 6.0 (about 900K SNPs).

Finally, the fetal fraction was measured using the genotyped SNP information of pregnant women and a linear regression analysis model (Y = 18.9X - 6.6). The linear regression analysis model used herein was a method disclosed in Jiang. P. et al., (2016) FetalQuantSD: accurate quantification of fetal DNA fraction by shallow-depth sequencing of maternal plasma DNA, npj Genomic Medicine, 1, 16013. The measured fetal fraction was used as a baseline fetal fraction.

**2. Statistical analysis**

Multivariate regression was used to determine the optimal parameters by training the data set by machine learning. In orderto implement the multivariate regression method, the elastic net method (Friedman, et al., J Stat Softw 2010;33 (1):1-22) was used. The elastic net method is a combination of the LASSO (least absolute shrinkage and selection operator) and the ridge method, and the performance speed is fast.

In order to improve the accuracy of fetal fraction estimation, chromosomes 13, 18, and 21 having a high incidence of trisomy among autosomal chromosomes were excluded. Each of the remaining 19 chromosomes was assigned to the constant bin size, and the characteristics of the read-count ratio and the characteristics the read-size ratio in each bin were extracted and used.

Specifically, the DNA readw were assigned to each chromosome of the reference genome, and then a locally weighted scatterplot smoothing (LOWESS) regression analysis was performed according to the following equation to reduce the bias of the GC content (Kim, et al., BMC medical genomics 2016;9 (1):22):

$$Rf_{ij'} = RC_{ij} / \sum_{j=1}^{j=K} RC_{ij} \qquad \text{(Equation 1).}$$

In Equation 1, $RC_{ij}$ is the GC-adjusted read count in autosomal chromosome bin j of sample i, and K is the maximum value of the bin divided in the autosomal chromosome. $Rf_{ij'}$ is the normalized value of $RC_{ij}$ in autosomal chromosome bin j of sample i.

On the other hand, since the distribution of the size of the fetal DNA fragments and the distribution of the size of the maternal DNA fragments are different from each other (Yu, et al., Proc Natl Acad Sci U S A 2014;111 (23):8583-8588), the size ratio of the DNA fragments in each bin of the autosomal chromosome is shown by the following equation:

Read-size ratio (SR) = number of DNA fragments smaller than 150 bp / number of total DNA fragments (Equation 2).

In order to normalize the size ratio of the DNA fragments, the following equation was used:

$$Sf_{ij'} = SR_{ij} / \sum_{j=1}^{j=K} SR_{ij} \qquad \text{(Equation 3).}$$

In Equation 3, $SR_{ij}$ is the read-size ratio in autosomal chromosome bin j of sample i, and K is the maximum value of the bin divided in the autosomal chromosome. $Sf_{ij'}$ is the normalized value of $SR_{ij}$ in autosomal chromosome bin j of sample i.

The multivariate regression equation used for data training can be shown as follows:

$$Y = \sum_{i=0}^{i=K} \beta_i X_i + e \qquad \text{(Equation 4).}$$

In Equation 4, Y is the fetal fraction measured with the Y chromosome, $\beta 0$ is the intercept, $\beta_{1-K}$ is the regression coefficient, and K is the maximum value of the bin divided in the autosomal chromosome. $X_i$ is the normalized value of read-count ratio or read-size ratio in bin i ($X_i = Rf_{ij'}$ or $X_i = S_{fij'}$), and e is a residual standard deviation. The fitted predicted value for Equation 4 can be shown as the following equation:

$$Y' = \sum_{i=0}^{i=K} \beta_i X_i \qquad \text{(Equation 5).}$$

In order to estimate the intercept and coefficient, cv.glmnet (Version 2.0-16) that supports 10-fold cross validation was used in the R package.

**3. Need for personalized analysis of fetal fraction according to read count of sample**

SeqFF (Kim et al., Prenatal diagnosis, 2015, vol.35, pp.810-815) is a software that enables the calculation of fetal fraction by entering the read-count ratio in a constant bin divided by 50 kb using parameters generated using training data with a sample size of 25,312. Data sets obtained from pregnant women carrying male fetuses as described in Example 1.1 were used. The fetal fraction was measured while increasing the test sample size from 100 to 500, and a Pearson correlation was calculated by comparing the measured fetal fraction and the standard fetal fraction. The standard fetal fraction is the fetal fraction measured using the Y chromosome. A graph of the Pearson correlation according to the test sample size is shown in FIG. 2.

As shown in FIG. 2, the correlation distribution between the test sample size and the standard fetal fraction ranged from about 73% to about 81%. This was 10% lower than that claimed by the SeqFF program (93%). The reason for the difference may be attributed to the difference in the distribution of sample read counts. This was because that the median read count of the samples used in SeqFF development was 17.19 million (M) (minimum = 3.82M, maximum = 34.7M), whereas the median read count of the samples in FIG. 2 was 5.85 million (M) (minimum = 3.38M, maximum = 12.45M).

Therefore, since the read count distribution of each sample will be different from each other, it is necessary to separately obtain parameters suitable for the read count distribution of each sample. In addition, it is necessary to select autosomal chromosome bin most suitable for the read count distribution of each sample without limiting the chromosome bin to 50k.

## 4. Selection of optimal autosomal chromosome bin by read count

### (1) Correlation distribution using read count

First, it was assumed that there was no difference between male and female fetuses in the bin of autosomal chromosome (excluding chromosomes 13, 18, and 21) when selecting the optimal autosomal chromosome bin.

The Pearson correlation was computed using randomly selected 1,000 male fetal training samples and 117 male fetal test samples. A graph of the Pearson correlation according to the bin size is shown in FIG. 3. As shown in FIG. 3, the male fetal test sample in the 64,000 kb bin exhibited a Pearson correlation of about 93.6%.

Using parameters obtained from training with 1,000 male fetal training samples in the 64,000 kb bin, the Pearson correlation for 45 female fetal test samples was computed. Here, the fetal fraction of 45 female fetal samples was measured based on the SNP. A graph of the Pearson correlation according to the bin size is shown in FIG. 4A. As shown in FIG. 4A, the female fetal test sample in the 64,000 kb bin exhibited a Pearson correlation of about 28%, yielding results completely different from the male fetal results. Considering this, unlike the assumption that there is no difference between male and female fetuses in the autosomal chromosome bin, it was determined that there was a difference between male and female fetuses in the autosomal chromosome bin.

Whereas certain bins of the autosomal chromosome of the male fetus may be highly correlated with the fetal fraction of the male fetus, these bins may be weakly correlated with the fetal fraction of the female fetus. In order to eliminate such bias, the test sample was thus validated using the female fetal sample, even if the training sample was validated using the fetal fraction based on the Y chromosome, Here, the fetal fraction of the female fetal sample was measured based on the SNP.

Using 1000 male fetal training samples and 45 female fetal test samples, the Pearson correlation was computed by comparing the female fetal fraction measured by different autosomal chromosome divided bins with the SNP-based standard fetal fraction. Here, the fetal fraction of the male fetal training sample was measured based on the Y chromosome, and the fetal fraction of the female fetal test sample was measured based on the SNP. The Pearson correlation distributions in various bins when tested using 45 female fetal samples are shown in FIGs. 4A, 4B, and 4C. As shown in FIGs. 4A to 4C, it had the highest correlation at about 79.6% in the 300k bin.

SeqFF has an autosomal chromosome-divided bin of 50 kb. In order to compare the accuracy of measuring the fetal fraction with an autosomal chromosome-divided bins of 50 kb and 300 kb, the Pearson correlation was computed by increasing the training sample size from 2000 to 5000 and setting the chromosome bin size to 50 kb or 300 kb. The computed results are shown in FIG. 5. As shown in FIG. 5, the correlation of 300 kb bin was higher by about 1% to about 5% than that of 50 kb bin.

### (2) Correlation distribution using read size

In order to estimate the fetal fraction based on the read-size ratio, the Pearson correlation according to the bin size was computed using 1,000 male fetal training samples and 45 female fetal test samples. The computed results are shown in FIG. 6. As shown in FIG. 6, the 800 kb bin had the highest correlation at about 72.5%.

## 5. Correlation with increasing training sample size in optimal autosomal chromosome bin

### (1) Computation of fetal fraction by read count and read size

As described in Example 1.4, the optimal chromosome bin was screened using 1,000 male fetal training samples and 45 female fetal test samples.

When the read-count ratio was used, the 300 kb bin had the most optimal Pearson correlation (FIG. 4C). When the read-size ratio was used, the 800 kb bin had the most optimal Pearson correlation (FIG. 6). Considering this, the correlation distribution was confirmed when combining the fetal fractions measured by the characteristics of different DNA fragments in different bins at the same time while increasing the number of training samples.

The Pearson correlations for the fetal fraction calculated using the read count, the fetal fraction calculated using the read size, or the average value of the two fetal fractions, which is a combination of the read count and the read size, were computed when the autosomal chromosome-divided bin is 300 kb, when the training sample size is increased while using 45 female fetal test samples. Here, the fetal fraction of the female fetal test sample was measured based on the SNP. The computed results are shown in FIG. 7A. As shown in FIG. 7A, the Pearson correlation of the combined fetal fraction of the read count and the read size was relatively higher than that using only the read-count ratio by about 4% or more. When 45 female fetal test samples were tested with parameters obtained from training with 5,000 male fetal training samples, the correlation of about 89% was shown.

In addition, the Pearson correlations for the fetal fraction calculated using the read count, the fetal fraction calculated using the read size, or the average value of the two fetal fractions, which is a combination of the read count and the read size, were computed when 45 male fetal test samples were used. Here, the fetal fraction of the male fetal test sample was measured based on the SNP. The computed results are shown in FIG. 7B. As shown in FIG. 7B, as the training sample size was increased, the correlation of the combined fetal fraction was relatively higher than that using only the read-count ratio by about 4% or more. When 45 male fetal test samples were tested with parameters obtained from training with 5,000 male fetal training samples, the correlation of about 89.7% was shown.

Since the correlation of the fetal fraction of the female fetal test sample is smilar to the correlation of the fetal fraction of the male fetal test sample, when the autosomal chromosome divided bin is 300 kb, it was confirmed that there was no bias that depends on the characteristics of the male fetus in each autosomal chromosome bin.

### (2) Computation of fetal fraction according to training sample size and test sample size

Under the conditions as described in Example 1.5(1), the Pearson correlation distribution of the fetal fraction was computed when the male fetal test sample size and the training sample size were changed.

The Pearson correlation computed when using 900 male fetal test samples and increasing the training sample size is shown in FIG. 8A. As shown in FIG. 8A, as the training sample size was increased, the correlation of the combined fetal fraction was increased by 90% or more.

The correlation computed when using 5,000 training samples and increasing the test sample size is shown in FIG. 8B. As shown in FIG. 8B, the correlation was about 90% or more in all samples.

The correlation between the fetal fraction estimated using 5,000 training samples and 900 test samples and the Y chromosome-based fetal fraction is shown in FIG. 8C. As shown in FIG. 8C, it was confirmed that the predicted fetal fraction had a correlation of about 91% with the Y chromosome-based fetal fraction.

### Claims

1. A method of determining a fetal fraction in a biological sample of a pregnant woman, the method comprising:

   generating training data and test data by obtaining sequence information (reads) of a plurality of nucleic acid fragments from a biological sample of a pregnant woman;
   setting a chromosome bin divided by constant bin based on a reference chromosome;
   generating a parameter from the training data;
   measuring a fetal fraction from the test data using the generated parameter;
   selecting a bin having a high correlation between the measured fetal fraction and the standard fetal fraction by repeating the steps of generating the parameter and measuring the fetal fraction while increasing the bin size based on the reference chromosome;
   generating a second parameter having an increased correlation between the measured fetal fraction and the standard fetal fraction while increasing the size of the training data in the selected bin; and
   measuring a fetal fraction from the test data using the generated second parameter.

2. The method of claim 1, wherein the biological sample is blood, plasma, serum, urine, saliva, mucus, sputum, feces, tears, or a combination thereof.

3. The method of claim 1, wherein the biological sample comprises nucleic acids derived from a fetus.

4.  The method of claim 1, wherein the generation of test data by obtaining sequence information of a plurality of nucleic acid fragments comprises isolating cell-free DNAs (cfDNAs) from the biological sample.

5.  The method of claim 1, wherein a sequencing coverage of the obtained sequence information is in a range of 0.00001 to 3.5.

6.  The method of claim 1, wherein the bin is a bin set in units of 5 kb to 260,000 kb.

7.  The method of claim 1, wherein the training data and the test data are sequence information derived from fetal samples of the same sex or fetal samples of a different sex.

8.  The method of claim 1, wherein the parameter is a read count, a read size, or a combination thereof.

9.  The method of claim 1, wherein the generation of a parameter from the training data is the training of the training data by a machine learning method.

10. The method of claim 9, wherein the training of the training data is performed by a multivariate regression model, a deep learning algorithm, or a combination thereof.

11. The method of claim 10, wherein the method is performed using an open source software library of the R package cv.glmnet, Tensorflow, or a combination thereof.

12. The method of claim 1, wherein the generation of a parameter from the training data comprises measuring the fetal fraction of the training data; and generating a parameter from the measured fetal fraction.

13. The method of claim 1, wherein the generation of a parameter from the training data is performed according to the following multivariate regression equation:

$$Y = \sum_{i=0}^{i=K} \beta_i X_i + e ,$$

in the above equation,

Y is the fetal fraction measured based on the SNP in the case of a female fetus, and the fetal fraction measured based on the SNP or with the Y chromosome in the case of a male fetus,
$\beta_0$ is the intercept,
$\beta_{1-K}$ is the regression coefficient,
K is the maximum value of the chromosome bin of the autosomal chromosome,
$X_i$ is the normalized value of the read-count ratio or the read-size ratio in bin i, and
e is a residual standard deviation.

14. The method of claim 1, wherein the fetal fraction is an average value of the fetal fraction measured using the read count and the fetal fraction measured using the read size.

15. A method of determining a fetal fraction in a biological sample of a pregnant woman, the method comprising:

generating test data by obtaining sequence information of a plurality of nucleic acid fragments from a biological sample of a pregnant woman;
setting a chromosome bin in units of 100 kb to 900 Kb based on a reference chromosome;
generating a read count, a read size, or a combination thereof as a parameter from the training data; and
measuring a fetal fraction from the test data using the generated parameter.

16. A computer readable medium, in which a program to be applied for performing the method according to any one of claims 1 to 15 is recorded.

FIG. 1

---

**Step 1. Personalized screening with a small number of samples**

| Machine learning changing the bin size with read count | Machine learning changing the bin size with read size |
|---|---|

| Select the optimal bin size of read count | Select the optimal bin size of read size |

---

**Step 2. Computing accurate results with a large number of samples**

| Machine learning with the optimal bin size of read count | Machine learning with the optimal bin size of read size |
|---|---|

**Combine both results**

---

FIG. 2

FIG. 3

FIG. 4A

FIG. 4B

FIG. 4C

FIG. 5

FIG. 6

FIG. 7A

FIG. 7B

FIG. 8A

FIG. 8B

FIG. 8C

<table>
<tr><td colspan="2" align="center">**INTERNATIONAL SEARCH REPORT**</td><td>International application No.<br>**PCT/KR2019/015927**</td></tr>
</table>

**A. CLASSIFICATION OF SUBJECT MATTER**

*G16B 20/40(2019.01)i, G16B 30/00(2019.01)i, G16B 50/00(2019.01)i*

According to International Patent Classification (IPC) or to both national classification and IPC

**B. FIELDS SEARCHED**

Minimum documentation searched (classification system followed by classification symbols)
G16B 20/40; G06F 19/00; G06F 19/18; G06F 19/22; G16B 20/00; G16B 30/00; G16B 50/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: fetal fraction, sequence information, constant section, parameter, correlation, training data

**C. DOCUMENTS CONSIDERED TO BE RELEVANT**

| Category* | Citation of document, with indication, where appropriate, of the relevant passages | Relevant to claim No. |
|---|---|---|
| A | KR 10-2016-0022374 A (SEQUENOM, INC.) 29 February 2016<br>See the entire document. | 1-16 |
| A | KR 10-2017-0016393 A (VERINATA HEALTH, INC.) 13 February 2017<br>See the entire document. | 1-16 |
| A | KR 10-2015-0105314 A (THE CHINESE UNIVERSITY OF HONG KONG) 16 September 2015<br>See the entire document. | 1-16 |
| A | KR 10-2017-0036649 A (SK TELECOM CO., LTD.) 03 April 2017<br>See the entire document. | 1-16 |
| A | US 2017-0316150 A1 (SEQUENOM, INC.) 02 November 2017<br>See the entire document. | 1-16 |
| A | KR 10-2018-0123020 A (VERINATA HEALTH, INC.) 14 November 2018<br>See the entire document. | 1-16 |
| A | RAMAN, L. et al. WisecondorX: improved copy number detection for routine shallow whole-genome sequencing. Nucleic Acids Research. 2019, vol. 47, no. 4, pages 1605-1614, (Electronic publication) 19 December 2018<br>See the entire document. | 1-16 |

☐ Further documents are listed in the continuation of Box C.    ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 MARCH 2020 (17.03.2020) | **17 MARCH 2020 (17.03.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/015927**

| Patent document cited in search report | Publication date | Patent family member | Publication date |
|---|---|---|---|
| KR 10-2016-0022374 A | 29/02/2016 | AU 2014-284180 A1 | 11/02/2016 |
| | | BR 112015032031 A2 | 25/07/2017 |
| | | CA 2915628 A1 | 24/12/2014 |
| | | CN 105473741 A | 06/04/2016 |
| | | DK 3011051 T3 | 23/04/2019 |
| | | EP 3011051 A1 | 27/04/2016 |
| | | EP 3011051 B1 | 30/01/2019 |
| | | EP 3540076 A1 | 18/09/2019 |
| | | ES 2721051 T3 | 26/07/2019 |
| | | HK 1223656 A1 | 04/08/2017 |
| | | JP 2016-533173 A | 27/10/2016 |
| | | JP 2018-196389 A | 13/12/2018 |
| | | JP 6473744 B2 | 20/02/2019 |
| | | MX 2015016911 A | 21/06/2016 |
| | | PL 3011051 T3 | 31/07/2019 |
| | | PT 3011051 T | 27/03/2019 |
| | | RS 58599 B1 | 31/05/2019 |
| | | SI 3011051 T1 | 31/05/2019 |
| | | US 2015-0005176 A1 | 01/01/2015 |
| | | WO 2014-205401 A1 | 24/12/2014 |
| KR 10-2017-0016393 A | 13/02/2017 | AU 2015-266665 A1 | 22/12/2016 |
| | | CA 2950596 A1 | 03/12/2015 |
| | | CN 106795558 A | 31/05/2017 |
| | | EP 3149199 A1 | 05/04/2017 |
| | | IL 249095 A | 31/01/2017 |
| | | JP 2017-524374 A | 31/08/2017 |
| | | US 10318704 B2 | 11/06/2019 |
| | | US 2016-0019338 A1 | 21/01/2016 |
| | | US 2019-0318805 A1 | 17/10/2019 |
| | | WO 2015-184404 A1 | 03/12/2015 |
| KR 10-2015-0105314 A | 16/09/2015 | AU 2014-205038 A1 | 16/07/2015 |
| | | AU 2014-205038 A8 | 13/08/2015 |
| | | AU 2014-205038 B2 | 27/07/2017 |
| | | AU 2017-228558 A1 | 05/10/2017 |
| | | AU 2017-228558 B2 | 30/05/2019 |
| | | AU 2017-228558 C1 | 03/10/2019 |
| | | CA 2895206 A1 | 17/07/2014 |
| | | CN 105051209 A | 11/11/2015 |
| | | EP 2943592 A1 | 18/11/2015 |
| | | HK 1210811 A1 | 06/05/2016 |
| | | HK 1213024 A1 | 24/06/2016 |
| | | JP 2016-511632 A | 21/04/2016 |
| | | JP 2018-075013 A | 17/05/2018 |
| | | JP 6251289 B2 | 20/12/2017 |
| | | KR 10-2019-0019219 A | 26/02/2019 |
| | | KR 10-2019-0122909 A | 30/10/2019 |
| | | KR 10-2038125 B1 | 29/10/2019 |

Form PCT/ISA/210 (patent family annex) (January 2015)

INTERNATIONAL SEARCH REPORT
Information on patent family members

International application No.

**PCT/KR2019/015927**

| Patent document cited in search report | Publication date | Patent family member | Publication date |
|---|---|---|---|
| | | SG 10201807047 A | 27/09/2018 |
| | | SG 11201504795 A | 30/07/2015 |
| | | US 2014-0195164 A1 | 10/07/2014 |
| | | WO 2014-107765 A1 | 17/07/2014 |
| KR 10-2017-0036649 A | 03/04/2017 | KR 10-1907650 B1 | 12/10/2018 |
| | | WO 2017-051996 A1 | 30/03/2017 |
| US 2017-0316150 A1 | 02/11/2017 | AU 2015-330734 A1 | 20/04/2017 |
| | | CA 2964158 A1 | 14/04/2016 |
| | | EP 3204512 A1 | 16/08/2017 |
| | | JP 2018-500876 A | 18/01/2018 |
| | | WO 2016-057901 A1 | 14/04/2016 |
| KR 10-2018-0123020 A | 14/11/2018 | AR 107192 A1 | 28/03/2018 |
| | | AU 2016-391100 A1 | 27/09/2018 |
| | | AU 2016-391100 B2 | 07/03/2019 |
| | | AU 2019-203491 A1 | 06/06/2019 |
| | | BR 112018015913 A2 | 22/01/2019 |
| | | BR 112018015913 B1 | 03/12/2019 |
| | | CA 3013572 A1 | 10/08/2017 |
| | | CN 108884491 A | 23/11/2018 |
| | | DK 3202915 T3 | 24/06/2019 |
| | | EA 201891580 A1 | 31/01/2019 |
| | | EP 3202915 A1 | 09/08/2017 |
| | | EP 3202915 B1 | 20/03/2019 |
| | | EP 3517626 A1 | 31/07/2019 |
| | | IL 260938 A | 20/09/2018 |
| | | KR 10-2019-0132558 A | 27/11/2019 |
| | | KR 10-2049191 B1 | 26/11/2019 |
| | | NZ 745637 A | 31/05/2019 |
| | | SG 11201806595 A | 27/09/2018 |
| | | TW 201805429 A | 16/02/2018 |
| | | TW 201930598 A | 01/08/2019 |
| | | TW I661049 B | 01/06/2019 |
| | | US 10095831 B2 | 09/10/2018 |
| | | US 2017-0220735 A1 | 03/08/2017 |
| | | US 2019-0065676 A1 | 28/02/2019 |
| | | WO 2017-136059 A1 | 10/08/2017 |
| | | ZA 201805753 B | 24/04/2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIANG P et al.** *Bioinformatics,* 2012, vol. 28, 2883-2890
- **HUDECOVA I et al.** *PloS one,* 2014, vol. 9, p.e88484
- **KIM et al.** *Prenatal diagnosis,* 2015, vol. 35, 810-815
- **JOHANSEN P et al.** *Prenatal diagnosis,* 2016, vol. 36, 530-536
- **HUDECOVA et al.** *PLoS One,* 2014, vol. 9 (2), e88484
- **JIANG. P et al.** FetalQuantSD: accurate quantification of fetal DNA fraction by shallow-depth sequencing of maternal plasma DNA, npj. *Genomic Medicine,* 2016, vol. 1, 16013
- **FRIEDMAN et al.** *J Stat Softw,* 2010, vol. 33 (1), 1-22
- **KIM et al.** *BMC medical genomics,* 2016, vol. 9 (1), 22
- **YU et al.** *Proc Natl Acad Sci U S A,* 2014, vol. 111 (23), 8583-8588